Europäisches Patentamt

European Patent Office

Office européen des brevets

(19)

(11) Numéro de publication : **0 043 309**
**B1**

(12) **FASCICULE DE BREVET EUROPÉEN**

(45) Date de publication du fascicule du brevet :
**11.04.84**

(21) Numéro de dépôt : **81400985.8**

(22) Date de dépôt : **19.06.81**

(51) Int. Cl.³ : **C 07 C 45/00**, C 07 C 49/08,
C 07 C 49/10, B 01 J 25/00

(54) **Procédé pour la mise en oeuvre de la fabrication d'une cétone par déshydrogénation d'un alcool secondaire.**

(30) Priorité : 26.06.80 FR 8014290
19.01.81 FR 8101018

(43) Date de publication de la demande :
06.01.82 Bulletin 82/01

(45) Mention de la délivrance du brevet :
11.04.84 Bulletin 84/15

(84) Etats contractants désignés :
AT BE CH DE GB IT LI NL

(56) Documents cités :
BE-A-   661 221
DE-C-   826 134
FR-A- 1 202 893
FR-A- 2 074 029
GB-A-   908 436
CHEMICAL ABSTRACTS, vol. 92, mai 1980, no. 19,
page 562, réf. no. 163525p, Columbus, Ohio, US, D.V.
SOKOL'SKII et al.: "Dehydrogenation of isopropyl
alcohol on Raney nickel catalysts, modified by copper and titanium additives, and without additives"

(73) Titulaire : **INSTITUT FRANCAIS DU PETROLE**
**4, Avenue de Bois-Préau**
**F-92506 Rueil-Malmaison Cédex (FR)**

(72) Inventeur : **Bournonville, Jean-Paul**
**5, rue des Champs Roger**
**F-78400 Chatou (FR)**
Inventeur : **Snappe, Roger**
**32, rue des Hauts Closeaux**
**F-92310 Sèvres (FR)**
Inventeur : **Miquel, Jean**
**5, rue Fragonard**
**F-75017 Paris (FR)**
Inventeur : **Martino, Germain**
**Bâtiment Condé 80, avenue F. Lefebvre**
**F-78300 Poissy (FR)**

Procédé pour la mise en œuvre de la fabrication d'une cétone par déshydrogénation d'un alcool
secondaire

La déshydrogénation de l'isopropanol, du butanol-2 et du cyclohexanol en acétone, méthyléthylcétone et cyclohexanone respectivement, peut être réalisée industriellement en phase vapeur, à une température relativement élevée (300-550 °C), en présence d'un catalyseur de déshydrogénation. Le catalyseur peut être à base d'un métal lourd appartenant à l'un des groupes I, II, VI, VII et VIII de la classification périodique des éléments, et plus particulièrement le métal peut être le cuivre, le magnésium, le nickel, le zinc, etc... ces métaux étant parfois additionnés d'autres métaux ou de leurs dérivés, tels que l'étain et le plomb, par exemple. La pression opératoire est en général faible et, souvent, diffère peu de la pression atmosphérique.

La nécessité de travailler en phase vapeur à température relativement élevée et à faible pression est la cause principale d'un certain nombre d'inconvénients :

1. apport important d'énergie calorifique à un niveau thermique élevé.

2. désactivation du catalyseur par diminution de la surface active qui résulte du grossissement des grains par réagglomération.

3. désactivation du catalyseur par dépôt de « coke » qui implique une régénération du catalyseur par combustion vers 500 °C à l'aide d'un mélange oxygène-azote à 2 % par exemple en oxygène, tous les dix jours dans certains cas.

4. conversion non totale de l'alcool, qui nécessite un fractionnement des effluents de réaction puis un recyclage de l'alcool non transformé.

5. existence de réactions parasites diminuant le rendement global et la pureté de l'hydrogène produit. Ces réactions sont par exemple :

a) la formation de produits de dégradation tels que le méthane et l'oxyde de carbone.

b) la déshydratation de l'alcool en oléfine ou en cyclooléfine, dont les réactions de condensation avec la cétone formée peuvent conduire à des produits analogues au diacétone alcool et, ultérieurement à l'oxyde de mésityle.

6. utilisation d'un appareillage peu commode à employer en raison des faits suivants :

— disposition du catalyseur dans des tubes de four où la régulation thermique est délicate

— démontage périodique pour le nettoyage ou le changement de catalyseur.

La mise en œuvre d'un procédé selon lequel la réaction s'effectue en phase liquide a permis d'éviter la plupart des inconvénients précédemment mentionnés.

Dans l'art antérieur, il a été établi d'une part que l'élimination de l'hydrogène était indispensable pour déplacer l'équilibre dans le sens de la transformation de l'alcool en cétone, thermodynamiquement défavorisée à basse température et d'autre part que l'évacuation de la cétone du milieu réactionnel, au fur et à mesure de sa formation, facilite le maintien d'une vitesse de réaction satisfaisante. La deuxième condition est réalisée en ajoutant au milieu réactionnel certains solvants qui ne réagissent pas sous les conditions opératoires utilisées et permettent de conduire la réaction à une température supérieure à la température de conduire la réaction à une température supérieure à la température d'ébullition de la cétone, à la pression utilisée.

Dans l'art antérieur, on a ainsi choisi des solvants qui possédaient notamment les caractéristiques suivantes :

— avoir un point d'ébullition élevé, supérieur au point d'ébullition de l'alcool à transformer.

— ne subir aucune transformation ni dégradation dans le milieu réactionnel.

— avoir un coefficient d'adsorption sur le catalyseur beaucoup plus faible que celui de l'alcool à transformer.

On peut citer un certain nombre de composés qui pouvaient ainsi être utilisés comme solvants : des hydrocarbures naphténiques lourds tels que le décahydronaphtalène, des hydrocarbures paraffiniques lourds, des hydrocarbures naphténiques possédant au moins une chaîne paraffinique tels que l'exadécyldécahydronaphtalène ou des mélanges des hydrocarbures précédents tels qu'on les trouve dans les fractions lourdes pétrolières.

Néanmoins un tel procédé en phase liquide est malgré tout très désavantageux par rapport au procédé classique en phase gazeuse. En effet, dans le procédé en phase liquide, la température réactionnelle dépasse rarement les 150 °C et, par conséquent, la conversion est très inférieure à celle obtenue par le procédé en phase gazeuse à 400 °C : la conversion est d'environ 25 % pour la fabrication de l'acétone et 10 % environ pour celle de la méthyléthylcétone, ce qui nécessite un recyclage très impossible d'éviter totalement un entraînement du solvant, ce qui impose un surdimensionnement au niveau du fractionnement. L'existence de réactions parasites d'hydrogénolyse productrices d'hydrocarbures légers tels que le méthane, l'éthane, le propane et le butane ne permet pas, au procédé en phase liquide, d'obtenir une sélectivité de 100 % : la pureté de l'hydrogène produit est de 98 % environ à une température voisine de 150 °C. L'élévation de la température qui favorise les réactions d'hydrogénolyse et l'évaporation du solvant ne semble pas être une bonne solution.

Or, on a maintenant découvert qu'il est possible d'opérer à une température suffisamment élevée, en phase liquide, à condition d'utiliser des solvants d'un type particulier, moins volatils que ceux employés auparavant et à condition également de mettre en œuvre des catalyseurs particuliers qui permettent d'obtenir une activité convenable tout en présentant une activité hydrogénolysante limitée.

Le solvant conforme à l'invention est constitué essentiellement d'au moins un hydrocarbure paraffinique contenant 12 à 20 atomes de carbone par molécule. De préférence, on utilise un mélange d'hydrocarbures paraffiniques, par exemple toute coupe saturée lourde : de préférence le point d'ébullition initial est supérieur à 240 °C à pression atmosphérique (0,1 MPa). Ce solvant ne doit contenir sensiblement aucun hydrocarbure cycloparaffinique ou cyclooléfinique. D'une façon générale la teneur de ce solvant en hydrocarbures aromatiques ou générateurs d'hydrocarbures aromatiques, dans les conditions opératoires de réaction, doit être inférieure à 1 000 p.p.m. (exprimée en benzène) et de préférence inférieure à 300 p.p.m. De même la teneur en soufre doit rester inférieure à 300 p.p.m. De même la teneur en soufre doit rester inférieure à 500 p.p.m. et de préférence à 200 p.p.m.

Un solvant de même type que le nôtre a été proposé dans le brevet français n° 2 074 029 mais dans ce document la nécessité d'utiliser un solvant ayant de faibles concentrations en soufre et hydrocarbures aromatiques n'a pas été notée. En outre, dans ce document, le catalyseur utilisé ne renferme aucun métal additionnel ainsi qu'il sera expliqué ci-dessous.

Des teneurs en hydrocarbures aromatiques ou générateurs d'hydrocarbures aromatiques et en soufre plus élevées que les spécifications fixées ne nuisent pas à la pureté de l'hydrogène (car elles inhibent également la réaction d'hydrogènolyse) mais provoquent une baisse sensible de la conversion. Les « générateurs d'hydrocarbures aromatiques » sont essentiellement les cycloparaffines et cyclooléfines.

La réaction est effectuée sous une pression généralement voisine de la pression atmosphérique, à une température comprise entre environ 170 et 230 °C mais de préférence entre 180 et 220 °C et plus particulièrement entre 185 et 210 °C. L'adoption de ce type de solvant et l'augmentation de la température permettent ainsi d'élever de 20 à 40 % la conversion par rapport à un procédé effectué vers 150 °C seulement.

Cependant il est connu que l'élévation de la température se traduit par une augmentation notable de l'hydrogénolyse et, par conséquent, par une diminution de la pureté de l'hydrogène produit. L'utilisation d'un catalyseur correctement choisi va permettre de pallier à ces inconvénients et d'assurer l'obtention d'une pureté molaire d'hydrogène de 97,5 % à 98 %.

Le catalyseur utilisé dans la présente invention est de type Nickel de Raney, c'est-à-dire qu'il contient du Nickel de Raney ou tout autre composé équivalent au Nickel de Raney où le nickel serait remplacé, au moins en partie, par un métal déshydrogénant traditionnel appartenant généralement au groupe VIII de la classification périodique des éléments, par exemple le cobalt, le platine, le rhodium etc... Mais (et ceci constitue la première technique de l'invention) il est impératif d'ajouter à ce catalyseur de type Nickel de Raney au moins un métal additionnel ou composé d'un métal additionnel, choisi dans le groupe constitué par le cuivre, l'argent, l'or, l'étain, le germanium, le plomb, le zinc, le cadmium et l'indium. L'ajout de ce métal additionnel provoque une diminution sensible de la réaction parasite d'hydrogénolyse. Le brevet français n° 2 074 029 n'utilise aucun métal additionnel.

L'introduction d'au moins un métal additionnel peut être réalisée soit au moment de la préparation de l'alliage de type Raney soit par addition directe à l'alliage de type Nickel de Raney déjà préparé et en suspension dans un liquide, d'un composé du métal choisi soluble dans la phase liquide contenant l'alliage de type Nickel de Raney.

La quantité de métal additionnel à ajouter est comprise généralement entre 0,1 et 10 % en poids (exprimé en élément métal) et de préférence entre 0,2 et 6 % en poids par rapport à la masse de type Nickel Raney utilisée. On obtient ainsi un catalyseur renfermant les quantités indiquées (0,1 à 10 % ou mieux 0,2 à 6 % en poids) de métal additionnel. La diminution de l'activité hydrogénolysante se traduit par une augmentation importante de la sélectivité, par la production d'un hydrogène de pureté correcte car il contient des teneurs faibles en hydrocarbures légers, produits de l'hydrogénolyse (méthane, éthane, etc...) et également par une durée de vie du catalyseur beaucoup plus longue.

Mais on a également découvert (et ceci constitue la deuxième technique de l'invention) qu'il était très avantageux d'introduire le ou lesdits métaux additionnels non pas comme indiqué ci-dessus mais par une autre méthode consistant à le ou les injecter en solution dans la zone de réaction, à la température réactionnelle, le catalyseur, en suspension dans le solvant de réaction, du type Nickel de Raney ayant été préalablement introduit dans la zone de réaction. Une méthode d'introduction du ou des métaux additionnels consiste à introduire le ou les composés de ces métaux en solution dans le solvant de réaction en quantité suffisante pour obtenir la teneur souhaitée en métal additionnel. Ensuite commence l'introduction de la charge, c'est-à-dire de l'alcool secondaire. Une autre méthode, généralement préférée, consiste à introduire le ou les métaux additionnels en solution dans l'alcool secondaire ou dans une partie de celui-ci jusqu'à obtention de la teneur désirée en métal additionnel.

L'addition du métal additionnel (conformément à ladite deuxième technique) se fait donc par injection dans le milieu réactionnel d'une solution d'au moins un composé d'un métal que l'on appelera arbitrairement composé organométallique dudit métal additionnel. Il convient donc

a) que la solubilité du ou des composés organométalliques utilisés dans le milieu réactionnel et/ou le solvant utilisé soit suffisante pour pouvoir ajouter la quantité de métal additionnel nécessaire à l'obtention d'une conversion et d'une pureté d'hydrogène élevées.

3

b) que la réactivité de ces composés organométalliques avec l'alcool à déshydrogéner soit sensiblement nulle.

Le ou les composés organométalliques utilisés ainsi pour la préparation du catalyseur sont choisis de préférence dans le groupe constitué par :

a) des alkylmétaux des métaux dits additionnels, dont le radical alkyle une structure paraffinique ou cycloparaffinique et comprend de 1 à 10 atomes de carbone mais de préférence de 3 à 6 atomes de carbone par molécule. On citera à titre d'exemples : le diéthylcadmium, le dibutylcadmium, le diméthylcadmium, le diéthylzinc, le dibutylzinc, le triméthylindium, le tétrapropylgermanium, le tétrabutylgermanium, le tétrabutylétain, le diméthyléthylpropylétain, le diméthyldiéthylétain, le tétraméthylplomb, le tétraéthylplomb, l'éthylargent.

b) les arylmétaux des métaux dits additionnels.

On citera à titre d'exemple: le diphénylétain, le diphénylgermanium, le triphénylbenzylplomb, le tétraphénylplomb, le diphénylzinc, le triphénylindium.

c) les alkylaryl métaux ou aryl alkyl métaux des métaux additionnels où le radical alkyl est défini comme en (a). On citera à titre d'exemple : le diéthyldiphénylétain, le diéthyldiphénylgermanium, le diéthylphénylindium, le méthylphénylzinc, le tétrabenzylétain et le tétrabenzylgermanium.

d) les acétylacétonates des métaux dits additionnels.

On citera à titre d'exemples : l'acétylacétonate de cuivre, l'acétylacétonate de cadmium, l'acétylacétonate de zinc, l'acétylacétonate d'indium.

e) les sels métalliques des métaux dits additionnels et des acides organiques dont la chaîne hydrocarbonée contient de 1 à 6 atomes de carbone et plus particulièrement de 1 à 4 atomes de carbone par molécule. On citera à titre d'exemples : l'acétate, le formiate et le butyrate de cuivre, l'acétate d'argent, l'acétate de zinc, l'acétate d'étain, l'acétate de plomb, l'acétate d'or, composés solubles dans l'alcool utilisé aux concentrations utilisées.

La quantité de métal à ajouter est comprise en général entre 0,1 et 10 % mais plus particulièrement entre 0,3 et 5 % en poids (exprimée en élément métal), de préférence entre 0,5 et 4 %, en poids, par rapport à la masse de nickel type Raney utilisée. En conséquence l'addition, dans le milieu réactionnel, au début de la réaction, du composé ou des composés organométalliques des métaux additionnels, se poursuivra jusqu'à ce que la teneur choisie ait été obtenue. (Le pourcentage de métal additionnel sur le catalyseur peut être déterminé à l'issue de la réaction lorsqu'on récupère le catalyseur utilisé ; un prélèvement dans le milieu réactionnel permet de vérifier qu'il n'y a pas de présence détectable de métal additionnel dans le milieu liquide).

Un des avantages de l'injection du composé organométallique en solution dans le solvant et/ou dans la charge d'alcool (deuxième technique) par rapport à l'utilisation d'un alliage de type Raney additionné d'un métal dit additionnel (première technique) est que généralement, les quantités totales de métal additionnel qu'il est nécessaire de déposer sur les grains de la masse catalytique sont moins élevées que les quantités totales de métal additionnel qu'il convient d'introduire dans la masse catalytique conformément à ladite première technique de l'invention. Cela pourrait être dû, peut-être, à ce que l'action du métal additionnel se situerait directement au niveau de la surface catalytique. Ainsi, tout le métal additionnel ajouté participerait à l'amélioration des performances du catalyseur, alors que dans le cas de l'addition, à l'alliage du type Raney, du métal additionnel conformément à la première technique, seule une fraction de ce métal additionnel interviendrait au niveau de la surface catalytique active, l'autre partie restant sous forme alliée avec le nickel dans la masse des grains de catalyseur.

Il est parfois préférable, pour la deuxième technique, d'opérer en présence d'hydrogène lors de l'ajout du métal additionnel en raison de la formation de sous-produits qui résultent :

a) de la réaction des composés organométalliques avec le Nickel de Raney puis

b) de leur décomposition ultérieure. L'hydrogène présent permet ainsi de libérer des radicaux hydrocarbonés qui, après recombinaison avec l'hydrogène présent, conduisent, entre autres, à des hydrocarbures facilement éliminés par distillation dans les conditions de fonctionnement de l'unité. L'hydrogène, nécessaire à l'élimination des sous-produits, est avantageusement l'hydrogène produit au cours de la déshydrogénation de l'alcool secondaire. Cela explique pourquoi, de façon préférée, le composé du métal additionnel est introduit en solution dans l'alcool à déshydrogéner.

Néanmoins l'injection du composé organométallique du métal additionnel peut se faire, non pas en solution dans la charge d'alcool à déshydrogéner, mais directement dans le solvant de réaction contenant le catalyseur de type Nickel de Raney. Il est avantageux d'injecter en même temps de l'hydrogène en provenance de toute source adéquate, afin d'effectuer la fixation du métal additionnel sur le catalyseur de type Nickel de Raney en présence d'hydrogène. L'injection de l'hydrogène peut se faire à un débit compris entre 0,5 et 2 litres par heure et par gramme de catalyseur du type Nickel de Raney.

L'utilisation du catalyseur conformément à ladite deuxième technique, va se traduire par une diminution de l'activité hydrogénolysante et, donc, par une augmentation de la sélectivité (diminution de la quantité de produits de dégradation formés).

Le procédé selon l'invention (avec utilisation de la première ou de la deuxième technique) est applicable à la fabrication de toute cétone dont le point d'ébullition, à pression atmosphérique (environ 0,1 MPa), est inférieur à la température réactionnelle choisie, cette température maximum étant de l'ordre de 220 °C voire 230 °C. En bref la température réactionnelle est comprise entre 170 et 230 °C ; lorsqu'on opère conformément à la deuxième technique, on préfère opérer entre 195 et 210 °C.

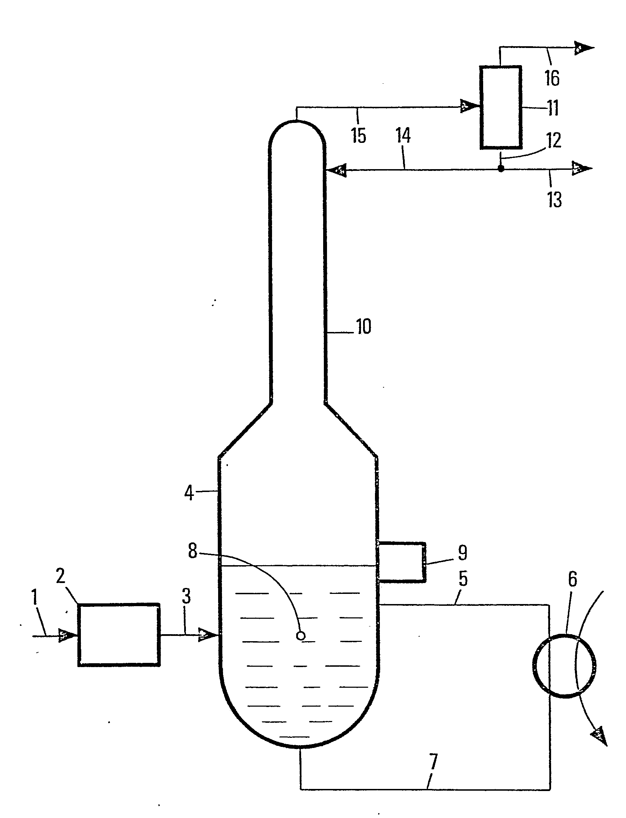

La préparation, selon l'invention, de cétones à partir d'alcools secondaires, en phase liquide, s'effectue, de préférence, avec élimination, par distillation, de la cétone produite, au fur et à mesure de sa formation ; cette élimination est effectuée dans tout appareillage convenable, mais de préférence, on opère avec un appareillage schématisé sur la figure unique.

L'alcool secondaire, que l'on désire transformer en cétone, est introduit dans le réacteur 4 par le conduit 1, par le système de pompage 2 et par le conduit 3. Ce réacteur 4 renferme le solvant et le catalyseur. Le réacteur 4 est muni d'un système d'agitation, schématisé par 5, 6 et 7, d'un dispositif 8 de mesure de la température, et d'un régulateur de niveau 9. Le chauffage s'effectue dans l'échangeur 6.

Le réacteur 4 renferme dans sa partie supérieure, ou est surmonté, d'un dispositif de fractionnement 10 dont l'efficacité règle la pureté du produit obtenu. Les vapeurs de la cétone produite, arrivant en tête de colonne, sont dirigées par la conduite 15 et sont condensées dans le condenseur 11. Le condensat est soutiré par la ligne 12. Une partie du condensat est recueillie par la ligne 13 et une autre partie est envoyée vers le haut de la colonne de fractionnement 10 par la conduite 14 afin de régler le taux de reflux. Dans la ligne 16, on récupère la fraction gazeuse riche en hydrogène.

Les exemples suivants, non limitatifs, illustrent l'invention ; dans ce qui suit, « conversion » signifie conversion en butanone (exemples 2 à 12) ou en acétone (exemple 13).

### Exemple 1

On prépare un catalyseur selon la première technique de l'invention en introduisant dans un récipient, muni d'un agitateur, 2 litres d'eau puis 500 g d'alliage Raney formé de 50 % en poids d'aluminium, de 47,5 % en poids de Nickel et de 2,5 % en poids de cuivre. On ajoute ensuite progressivement, en 3 heures, 1 litre d'une solution aqueuse à 750 g par litre d'hydroxyde de sodium, en ayant soin de maintenir la température à 70 °C environ. Après une heure d'agitation supplémentaire, on laisse décanter, on sépare la phase aqueuse et on lave le solide 4 fois à l'eau à la température ordinaire et le catalyseur, renfermant 5 % de cuivre en poids, est alors prêt à l'emploi. Pour préparer des catalyseurs renfermant par exemple de l'or, ou de l'argent ou du germanium ou de l'étain, il suffit de remplacer le cuivre par la quantité désirée du métal souhaité.

### Exemple 2

On se propose de fabriquer de la méthyléthylcétone, à partir de butanol-2.

Comme solvant, on utilise un solvant A défini ci-dessous :

Ce solvant A est constitué essentiellement d'hydrocarbures paraffiniques normaux possédant 12 à 16 atomes de carbone par molécule et possède les caractéristiques suivantes :

| | |
|---|---|
| — $d_4^{15}$ | 0,774 |
| — distillation PI | 250 °C |
| 50 % | 270 °C |
| PF | 300 °C |
| — hydrocarbures aromatiques (et générateurs) | 150 ppm |
| — soufre total | 100 ppm |

L'appareillage utilisé est un réacteur de 2 litres de capacité muni d'un système d'agitation efficace, d'un système de régulation et de contrôle de température et d'un système de fractionnement et de séparation conforme à la figure.

Les gaz et liquides obtenus sont mesurés et analysés par chromatographie en phase gazeuse.

Le réacteur contient le solvant A choisi pour conduire la réaction en phase liquide à la température désirée et pour maintenir le catalyseur, en suspension dans le milieu réactionnel. La charge que l'on veut transformer est introduite dans le réacteur au moyen d'une pompe doseuse.

Le butanol-2 (d = 0,808) est injecté dans le réacteur où se trouve le solvant A avec le catalyseur maintenu en suspension grâce à une bonne agitation ; la température du milieu réactionnel est 190 °C. Le butanol-2 est introduit à un pph = 38 kg de charge par kg de catalyseur et par heure. Le catalyseur utilisé est du nickel de Raney contenant 5 % en poids de cuivre et préparé comme indiqué à l'exemple 1.

Les conversions molaires (instantanées) obtenues au bout de 1,5 et 5 heures de marche sont respectivement de 43,2 % et 43,5 % et la pureté de l'hydrogène obtenu de 97,9 % et 97,6 % molaire.

Avec un catalyseur renfermant maintenant 0,15 % de cuivre, la pureté de l'hydrogène au bout de 1,5 et 5 heures, est respectivement 95,5 % et 95,3 %. Avec un catalyseur renfermant 0,25 % de cuivre, la pureté de l'hydrogène, au bout de 1,5 et 5 heures, passe respectivement à 97,1 % et 96,9 %.

### Exemple 3 (comparatif)

On répète l'exemple 2 dans les mêmes conditions, en remplaçant le catalyseur par du Nickel Raney dépourvu d'additif, c'est-à-dire dépourvu de cuivre.

Au bout de 1,5 et 5 heures de marche, la conversion molaire (instantanée) est respectivement de 43,6 % et 43,5 %. Mais la pureté de l'hydrogène obtenu n'est que de 89,2 % et 89,1 % molaire.

La comparaison des exemples 2 et 3 met en évidence l'effet inhibiteur sur l'activité hydrogénolysante apporté par l'addition, au Nickel de Raney, d'une certaine quantité de cuivre.

Exemple 4 (comparatif)

On répète l'exemple 2 en utilisant un solvant B non conforme à l'invention.
Le solvant B a la composition suivante :

— Paraffines (12 à 16 atomes de carbone par molécule)      57   % poids
— Cycloparaffines                                          50   % poids
— Aromatiques                                              2,9 % poids
— Oléfines                                                 0,1 % poids

Ses caractéristiques sont les suivantes ;

— $d_4^{15}$                                              0,815
— distillation PI                                          271 °C
    50 %                                                   283 °C
    PF                                                     303,5 °C
— soufre total                                             50 ppm

Les conversions molaires obtenues au bout de 1,5 heures et 5 heures de marche sont respectivement de 26,4 % et 19,2 %.

La pureté de l'hydrogène obtenu est respectivement de 90,5 % et 91,8 % molaire.

Ces résultats mettent en relief l'intérêt d'éviter l'emploi de solvants contenant d'autres hydrocarbures que les paraffines.

Exemple 5

On répète l'exemple 2, en utilisant des catalyseurs dans lesquels on a remplacé le cuivre par un métal qui est successivement l'étain, le germanium, l'argent et l'or.

La nature des catalyseurs et les résultats obtenus sont indiqués dans le tableau I

Tableau I

| Métal (% en poids) | Conversion molaire après : | | Pureté H₂ après : | |
|---|---|---|---|---|
| | 1,5 h | 5 h | 1,5 h | 5 h |
| 5   % Cu | 43,2 | 43,5 | 97,9 | 97,6 |
| 5   % Sn | 42,6 | 42,7 | 98,2 | 98,1 |
| 5   % Ge | 42,8 | 42,5 | 98,3 | 98,3 |
| 2,5 % Ag | 43,1 | 43,6 | 98,0 | 97,8 |
| 2,5 % Au | 43,4 | 43,4 | 97,9 | 98,0 |

Exemple 6

On répète l'exemple 2 en utilisant divers solvants A₁ à A₄ semblables au solvant A et s'en différenciant par les teneurs en hydrocarbures aromatiques et en soufre.

Le tableau II résume les résultats obtenus.

Tableau II

| Solvant | A | A₁ | A₂ | A₃ | A₄ |
|---|---|---|---|---|---|
| ppm hydrocarbures aromatiques | 150 | 280 | 380 | 950 | 1 100 |
| ppm soufre | 100 | 180 | 220 | 450 | 550 |
| Conversion molaire après 1,5 heure | 43,2 | 43,1 | 41,6 | 40,1 | 37,2 |

(Tableau II, suite)

| Solvant | A | A₁ | A₂ | A₃ | A₄ |
|---|---|---|---|---|---|
| Conversion molaire après 5 heures | 43,5 | 43,4 | 42,1 | 38,5 | 34,1 |
| Pureté de l'hydrogène obtenu (molaire) après 1,5 heure | 97,9 | 97,9 | 98,0 | 98,1 | 98,1 |
| Pureté de l'hydrogène obtenu (molaire) après 5 heures | 97,6 | 97,6 | 97,7 | 97,7 | 97,8 |

Exemple 7

On répète l'exemple 1 à diverses températures.
Les résultats sont rassemblés dans le tableau III

Tableau III

| Température (°C) | 240 | 220 | 190 | 180 | 175 | 150 |
|---|---|---|---|---|---|---|
| Conversion molaire après 1,5 heure | 35,8 | 42,7 | 43,2 | 42,6 | 40,9 | 31,5 |
| Pureté de l'hydrogène obtenu (molaire) après 1,5 heure | 97,9 | 97,9 | 97,9 | 97,9 | 97,9 | 98,0 |

En conclusion, on constate (essais comparatifs des tableaux II et III) qu'en n'opérant pas aux températures revendiquées ou avec le solvant revendiqué, il arrive que l'on obtienne une pureté de l'hydrogène légèrement supérieure à celle qu'on obtient en opérant conformément à l'invention mais alors, la conversion est nettement moins bonne que celle obtenue en opérant conformément à l'invention.

Il arrive aussi (exemple 3) qu'en omettant le métal additionnel, la conversion soit légèrement meilleure qu'avec l'emploi d'un métal additionnel. Mais cela est aux dépens de la pureté de l'hydrogène.

L'intérêt de l'invention est d'obtenir simultanément, par un choix critique de plusieurs paramètres, une conversion satisfaisante et une pureté convenable de l'hydrogène produit, donc une sélectivité correcte.

Exemple 8

On se propose de fabriquer la méthyléthylcétone à partir de butanol-2 ($d_4^{20}$ = 0,808) et en opérant conformément à ladite deuxième technique.

Comme solvant on utilise le solvant A défini à l'exemple 2.

L'appareillage utilisé est celui de l'exemple 2.

Les produits obtenus sont mesurés et analysés par chromatographie en phase gazeuse.

Une agitation efficace est indispensable pour mener la réaction en phase liquide et maintenir le catalyseur en suspension dans le milieu réactionnel.

Après stabilisation de la température de la zone de réaction (renfermant du solvant et du nickel de Raney en suspension : l'alliage Raney de départ utilisé ici est formé de 52 % en poids d'aluminium et de 48 % en poids du nickel) à la température de réaction choisie, on injecte une solution contenant l'alcool à déshydrogéner et le composé organométallique dans des proportions adéquates pour obtenir la teneur désirée en métal additionnel.

Dans le tableau IV sont portés les résultats obtenus après 5 heures d'injection de butanol-2 pour diverses quantités de tétrabutylétain injectées sous forme de solution à 10 % en volume dans l'alcool secondaire. Le temps zéro est arbitrairement assimilé à la fin de l'injection de la solution alcoolique de tétrabutylétain et correspond au début de l'injection de l'alcool pur. Les conditions opératoires sont les suivantes : température 180 °C, pph = 8 kg/kg de catalyseur/heure et pression voisine de la pression atmosphérique. La quantité de nickel de type Raney mise en œuvre est égale à 10 grammes. Le rendement en cétone par rapport à l'alcool converti est dans tous les cas supérieur à 99,5 %.

Il apparaît que l'addition d'étain provoque une légère baisse de conversion. Cependant cette légère baisse de conversion est compensée par une augmentation notable de la sélectivité, traduite par la pureté molaire de l'hydrogène formé. Mais l'intérêt de l'invention est précisément d'obtenir simultanément, par un choix critique de plusieurs paramètres, une conversion satisfaisante et une pureté convenable de l'hydrogène produit, donc une sélectivité correcte.

## Tableau IV

| Catalyseur | Conversion molaire (%) | Pureté molaire de l'hydrogène (%) |
|---|---|---|
| Nickel de Raney seul | 44,6 | 90,4 |
| + 0,34 % Sn | 43,4 | 94,1 |
| + 0,68 % Sn | 42,4 | 96,0 |
| + 1,36 % Sn | 41,4 | 97,6 |
| + 2,04 % Sn | 41,2 | 98,5 |
| + 2,72 % Sn | 40,5 | 99,1 |
| + 6,50 % Sn | 31,0 | 98,7 |
| + 10,0 % Sn | 14,0 | 99,0 |

(% Sn par rapport à la masse catalytique)

### Exemple 9

On répète l'exemple 8 à différentes températures. Deux catalyseurs sont comparés : le Nickel de Raney seul et le Nickel de Raney additionné de 2,72 % d'étain conformément à la technique de l'exemple 8. Les résultats sont consignés dans le tableau V. (Les conditions opératoires sont légèrement différentes de celles des exemples 2 et 3 ; on retrouve ici pour un Nickel Raney dépourvu de métal additionnel, des résultats sensiblement équivalents à ceux de l'exemple 3.

On note que c'est à partir d'environ 200 °C que l'action inhibitrice de l'étain se manifeste le plus : il en résulte une amélioration de la sélectivité par diminution de la production d'hydrocarbures légers (méthane, éthane, propane et butane) au profit de la méthyléthylcétone. Le rendement en cétone, dans tous les cas, par rapport à l'alcool converti, est supérieur à 99,5 %).

### Tableau V

| Température (°C) | Nickel de Raney | | Nickel de Raney + 2,7 % Sn | |
|---|---|---|---|---|
| | Conversion (%) | Pureté H$_2$ (%) | Conversion (%) | Pureté H$_2$ (%) |
| 160 | 30 | 97 | 28 | 99,7 |
| 180 | 44 | 90,5 | 41 | 99,0 |
| 200 | 57 | 83,3 | 53 | 97,1 |
| 210 | 64 | 79,0 | 59 | 96,0 |

(% Sn par rapport à la masse catalytique)

### Exemple 10

On répète l'exemple 8 en employant des catalyseurs de type Nickel de Raney auxquels on a ajouté un métal additionnel qui est successivement le cuivre, l'argent, l'or, le germanium, le plomb, l'indium et le zinc. les résultats sont rassemblés dans le tableau VI. L'addition de composés organométalliques permet donc de conserver une activité élevée et d'améliorer la sélectivité. (Dans le cas de l'emploi des acétates d'argent et d'or, insolubles dans le solvant, on a introduit ces acétates en solution dans l'alcool utilisé). Le rendement en cétone, dans tous les cas, par rapport à l'alcool converti, est supérieur à 99,5 %.

### Tableau VI

| Métal additionnel | | | Conversion Molaire (%) | Pureté Hydrogène % |
|---|---|---|---|---|
| Nature | Composé | % poids | | |
| Cuivre | Acétylacétonate | 2,5 | 40,8 | 98,9 |
| Argent | Acétate | 2,5 | 40,2 | 98,7 |
| Or | Acétate | 4,5 | 39,9 | 98,5 |
| Germanium | Tétrabutyl | 1,7 | 40,5 | 99,0 |
| Plomb | Tétraéthyl | 4,7 | 40,0 | 98,9 |
| Zinc | Acétylacétonate | 1,5 | 40,7 | 98,8 |
| Indium | Triméthyl | 2,6 | 41,0 | 98,6 |

(% poids de métal exprimé par rapport à la masse catalytique)

## Exemple 11

On opère dans les mêmes conditions que l'exemple 8 à la différence que le composé organométallique est introduit dans la zone de réaction en solution dans le solvant de réaction et que l'on introduit ainsi la quantité voulue de métal additionnel avent de commencer à introduire le butanol-2 (on a ainsi introduit 2,72 % d'étain dans la masse catalytique).

La conversion obtenue est égale à 39,4 % et la pureté molaire de l'hydrogène de 96,7 %. On rappelle que dans l'exemple 8, pur une teneur de 2,72 % en étain dans le catalyseur, on obtenait une conversion de 40,5 % et une pureté molaire de l'hydrogène de 99,1 %.

Ce mode opératoire d'injection de l'additif organométallique conduit donc à une conversion et une pureté d'hydrogène inférieures à celles obtenues après injection du composé organométallique en milieu alcoolique. Cette technique n'est donc intéressante que si l'on cherche essentiellement à éviter toute interaction entre l'alcool à deshydrogéner et le composé organométallique.

## Exemple 12

On répète l'exemple 8 avec un catalyseur C sur lequel, selon la deuxième technique de la présente invention, on a déposé 2,50 % en poids d'étain. Mais on opère également, conformément à ladite première technique sur deux catalyseurs $C_1$ et $C_2$ à base de Nickel de Raney dans lesquels, au moment de la préparation de l'alliage, on a incorporé respectivement 6,50 % et 2,50 % d'étain en poids.

Le tableau VII donne les résultats obtenus avec les trois catçlyseurs testés. On notera qu'avec les catalyseurs $C_1$ et $C_2$, il faut une teneur de 6,5 % en étain pour obtenir une comparaison valable avec le catalyseur C qui se contente d'une teneur de 2,5 % seulement en étain. Avec le catalyseur $C_2$, la teneur de 2,5 % en étain est encore légèrement insuffisante pour obtenir une pureté d'hydrogène excellente. Les rendements en cétone, par rapport à l'alcool converti, sont respectivement (avec les catalyseurs $C_1$, $C_2$ et C) de 95 %, 98,5 % et 99,5 %.

### Tableau VII

| Température (°C) | Catalyseur $C_1$ (6,5 % Sn) | | Catalyseur $C_2$ (2,5 % Sn) | | Catalyseur C (2,5 % Sn) | |
|---|---|---|---|---|---|---|
| | Conversion (%) | Pureté $H_2$ (%) | Conversion (%) | Pureté $H_2$ (%) | Conversion (%) | Pureté $H_2$ (%) |
| 180 | 42 | 97,9 | 43,2 | 94,1 | 41 | 99,0 |
| 190 | 42,6 | 97,9 | 46,2 | 92,0 | 46,5 | 98,0 |
| 200 | 42,3 | 97,8 | 50,1 | 90,1 | 52,7 | 97,6 |
| 210 | 42,5 | 97,7 | 56,2 | 88,0 | 58,5 | 97,1 |

L'avantage d'employer le catalyseur conforme à la deuxième technique par rapport à un catalyseur préparé à partir de l'alliage préalablement additionné d'étain (première technique) est plus net à une témpérarure, ici, voisine de 200-210 °C.

## Exemple 13

On se propose de fabriquer de l'acétone dans des conditions opératoires identiques à celles adoptées dans l'exemple 8, si ce n'est que l'on injecte de l'isopropanol à la place du butanol-2. Le catalyseur, à base de nickel de type Raney, contient 2,9 % en poids d'étain selon le processus de l'exemple 8.

La conversion obtenue après 5 heures est égale à 35 % (rendement en cétone : 99,5 %) et la pureté molaire de l'hydrogène produit est supérieure à 99 %. Dans les mêmes conditions, le Nickel de Raney seul (sans étain) permet une conversion égale à 38,5 % (rendement en cétone : 97 %) mais à une pureté en hydrogène égale à 90,6 %. L'ajout d'étain rend donc le catalyseur plus sélectif.

**Revendications**

1. Procédé pour la fabrication en phase liquide d'au moins une cétone par déshydrogénation d'au moins un alcool secondaire, caractérisé en ce que l'on opère à une température comprise entre environ 170° et 230 °C dans un solvant essentiellement constitué d'au moins un hydrocarbure paraffinique contenant 12 à 20 atomes de carbone par molécule, ledit solvant ne renfermant pas plus de 1 000 ppm (exprimé en benzène) d'hydrocarbures aromatiques ou de produits générateurs d'hydrocarbures aromatiques et pas plus de 500 ppm de soufre, et en présence d'un catalyseur de type nickel de Raney renfermant au moins un métal additionnel choisi dans le groupe constitué par le cuivre, l'argent, l'or, l'étain, le plomb, le zinc, le cadmium, l'indium et le germanium, la quantité de métal additionnel étant comprise entre 0,1 et 10 % en poids par rapport au catalyseur de type Nickel Raney.

2. Procédé selon la revendication 1 dans lequel la température est comprise entre 185° et 210 °C.

3. Procédé selon la revendication 1 dans lequel le solvant ne renferme pas plus de 300 ppm (exprimé en benzène) d'hydrocarbures aromatiques ou de produits générateurs d'hydrocarbures aromatiques, et pas plus de 200 ppm de soufre.

4. Procédé selon la revendication 1 dans lequel le catalyseur renferme 0,2 à 6 % en poids de métal additionnel.

5. Procédé selon la revendication 1 dans lequel la cétone produite est éliminée, par distillation, du milieu réactionnel au fur et à mesure de sa formation.

6. Procédé selon la revendication 1 pour la mise en œuvre d'une fabrication en phase liquide d'au moins une cétone, fabrication effectuée par déshydrogénation d'au moins un alcool secondaire en présence d'un catalyseur de type Nickel de Raney, le procédé de mise en œuvre étant caractérisé en ce que, dans la zone réactionnelle, préalablement chauffée à la température réactionnelle et renfermant au moins le catalyseur de type Nickel Raney en suspension dans ledit solvant de réaction, on introduit une solution d'un composé du métal, dit additionnel, en quantité suffisante pour que la masse catalytique renferme, (à l'issue du traitement de la masse catalytique par un composé du métal dit additionnel), en poids, 0,2 à 6 % (exprimé en élément métal) du métal dit additionnel, ladite introduction du composé d'un métal étant soit réalisée et achevée avant le début de l'introduction de l'alcool secondaire, soit a) réalisée en même temps que le début de l'alcool secondaire dans la zone réactionnelle puis b) poursuivie jusqu'à obtention du poids désiré en métal dans ledit catalyseur.

7. Procédé selon la revendication 6 dans lequel la température de la réaction de déshydrogénation de l'alcool secondaire est comprise entre 195 et 210 °C.

8. Procédé selon la revendication 6 dans lequel le composé du métal dit additionnel est choisi dans le groupe constitué par :

a) les alkylmétaux d'un métal additionnel dont le radical alkyle possède une structure paraffinique ou cycloparaffinique et comporte de 1 à 10 atomes de carbone par molécule,

b) les arylmétaux d'un métal additionnel,

c) les alkylarylmétaux ou les arylalkylmétaux d'un métal additionnel, où le radical alkyle est défini comme au § a) ci-dessus,

d) les acétylacétonates d'un métal additionnel, et

e) les sels métalliques d'un métal additionnel et des acides organiques renfermant 1 à 6 atomes de carbone par molécule.

9. Procédé selon la revendication 6 dans lequel ledit composé d'un métal est introduit en solution dans ledit solvant de réaction, avant le début de l'introduction de l'alcool secondaire.

10. Procédé selon la revendication 9, dans lequel on introduit également, en même temps que le composé d'un métal, de l'hydrogène avec un débit compris entre 0,5 et 2 litres par heure et par gamme du catalyseur du type Nickel de Raney.

11. Procédé selon la revendication 6 dans lequel ladite introduction du composé organométallique est réalisée en même temps que l'on commence à introduire l'alcool secondaire dans la zone réactionnelle, ledit composé organométallique étant introduit en solution dans l'alcool secondaire.

12. Procédé selon la revendication 6, dans lequel la teneur en poids en métal dit additionnel est comprise entre 0,5 et 4 %.

13. Procédé selon la revendication 6, dans lequel le métal additionnel est l'étain.

**Claims**

1. A process for manufacturing in the liquid phase at least one ketone by dehydrogenation of at least one secondary alcohol, characterized in that the operation is conducted at a temperature from about 170 °C to 230 °C in a solvent essentially consisting of at least one paraffinic hydrocarbon containing 12 to 20 carbon atoms per molecule, said solvent containing at most 1 000 ppm (expressed as benzene) of aromatic hydrocatbons or aromatic hydrocarbon generators and not more than 500 ppm of sulfur, and in the presence of a catalyst of the Raney nickel type containing at least one additional metal selected from the group consisting of copper, silver, gold, tin lead, zinc, cadmium, indium and germanium, the amount of the additional metal being from 0.1 to 10 % by weight with respect to the catalyst of the Raney nickel type.

2. A process according to claim 1 wherein the temperature is from 185 to 210 °C.

3. A process according to claim 1, wherein the solvent contains at most 300 ppm (expressed as benzene) of aromatic hydrocarbons or aromatic hydrocarbon generators and at most 200 ppm of sulfur.

4. A process according to claim 1, wherein the catalyst contains from 0.2 to 6 % by weight of additional metal.

5. A process according to claim 1, wherein the produced ketone is removed, by distillation, from the reaction medium as it is formed.

**0 043 309**

6. A process according to claim 1, for the manufacture in the liquid phase of at least one ketone by dehydrogenation of at least one secondary alcohol in the presence of a catalyst of the Raney nickel type, said process being characterized in that, in the reaction zone, previously heated to the reaction temperature and containing at least the catalyst of the Raney nickel type suspended in said reaction solvent, there is introduced a solution of a compound of the so-called additional metal, in such an amount that the catalyst mass contains (at the end of the treatment of the catalyst mass by means of a compound of said additional metal) from 0.2 to 6 % by weight (expressed as metal element) of said additional metal, said introduction of the metal compound being either effected and completed before ghe beginning of the introduction of the secondary alcohol or a) effected simultaneously with the beginning of the introduction of the secondary alcohol in the reaction zone and then b) continued up to the obtainment of the desired metal weight in said catalyst.

7. A process according to claim 6, wherein the reaction temperature of the secondary alcohol dehydrogenation is from 195 to 210 °C.

8. A process according to claim 6, wherein the so-called additional metal compound is selected from the group consisting of :

a) the alkyl metals of an addition metal whose alkyl radical has a paraffinic or cycloparaffinic structure and contains from 1 to 10 carbon atoms per molecule,

b) the aryl metals of an additional metal,

c) the alkylarylmetals or the arylalkylmetals of an additional metal, where the alkyl radical is defined as in paragraph a) above,

d) the acetylacetonates of an additional metal, and

e) the metal salts of an additional metal with organic acids containing from 1 to 6 carbon atoms per molecule.

9. A process according to claim 6, wherein said metal compound is introduced in solution in said reaction solvent before the beginning of the introduction of the secondary alcohol.

10. A process according to claim 9, also comprising the introduction, simultaneously with the metal compound, of hydrogen at a flow rate from 0.5 to 2 liters per hour and per gram of catalyst of the Raney nickel type.

11. A process according to claim 6, wherein said introduction of the organometallic compound is effected simultaneously with the introduction of the secondary alcohol in the reaction zone, said organometallic compound being introduced as a solution in the secondary alcohol.

12. A process according to claim 6, wherein the content by weight of said additional metal is from 0.5 to 4 %.

13. A process according to claim 6, wherein said additional metal is tin.

**Ansprüche**

1. Verfahren zur Herstellung mindestens eines Ketons durch Dehydrierung mindestens eines sekundären Alkohols in flüssiger Phase, dadurch gekennzeichnet daß man bei einer Temperatur zwischen etwa 170 und 230 °C in einem Lösungsmittel arbeitet, daß im wesentlichen aus mindestens einem paraffinischen Kohlenwasserstoff mit 12 bis 20 Kohlenstoffatomen pro Molekül besteht und nicht mehr als 1 000 ppm aromatische Kohlenwasserstoffe (ausgedrückt als Benzol) oder aromatische Kohlenwasserstoffe bildende Produkte und nicht mehr als 500 ppm Schwefel enthält, ferner in Anwesenheit eines Katalysators vom Typ Raney-Nickel, der mindestens ein zusätzliches Metall aus der Gruppe Kupfer, Silber, Gold, Zinn, Blei, Zink, Cadmium, Indium und Germanium enthält, wobei die Menge des zusätzlichen Metalls 0,1 bis 10 Gew.-% bezogen auf den Katalysator vom Typ Raney-Nickel beträgt.

2. Verfahren gemäß Anspruch 1, dadurch gekennzeichnet, daß die Temperatur 185 bis 210 °C beträgt.

3. Verfahren gemäß Anspruch 1, dadurch gekennzeichnet, daß das Lösungsmittel nicht mehr als 300 ppm aromatische Kohlenwasserstoffe (ausgedrückt als Benzol) oder aromatische Kohlenwasserstoffe bildende Produkte sowie nicht mehr als 200 ppm Schwefel enthält.

4. Verfahren gemäß Anspruch 1, dadurch gekennzeichnet, daß der Katalysator 0,2 bis 6 Gew.-% des zusätzlichen Metalls enthält.

5. Verfahren gemäß Anspruch 1, dadurch gekennzeichnet, daß das gebildete Keton durch Destillation aus dem Reaktionsmilieu entfernet wird, in dem Maße wie es sich bildet.

6. Verfahren gemäß Anspruch 1, zur Herstellung mindestens eines Ketons durch Dehydrierung mindestens eines sekundären Alkohols in flüssiger Phase in Gegenwart eines Katalysators vom Typ Raney-Nickel, dadurch gekennzeichnet, daß man in die Reaktionszone, die vorher auf die Reaktionstemperatur erhitzt wurde und mindestens den Katalysator vom Typ Raney-Nickel suspendiert in dem Reaktionslösungsmittel enthält, eine Lösung einer Verbindung des Zusatzmetalls in ausreichender Menge einleitet, so daß die katalytische Masse (nach Behandlung derselben mit einer Verbindung des Zusatzmetalls) 0,2 bis 6 Gew.-% (ausgedrückt als elementares Metall) des Zusatzmetalls enthält, wobei die Einleitung dieser Metallverbindung entweder vor Beginn der Zufuhr des sekundären Alkohols durchgeführt und beendet wird oder a) gleichzeitig mit der beginnenden Einführung des sekundären Alkohols in

11

die Reaktionszone durchgeführt und anschließend b) fortgesetzt, bis man die gewünschte Menge Metall im Katalysator erhalten hat.

7. Verfahren gemäß Anspruch 6, dadurch gekennzeichnet, daß die Reaktionstemperatur der Dehydrierung des sekundären Alkohols 195 bis 210 °C beträgt.

8. Verfahren gemäß Anspruch 6, dadurch gekennzeichnet, daß die Verbindung des Zusatzmetalls aus der folgenden Gruppe gewählt wird :

a) Metallalkyle eines Zusatzmetalls, wobei die Alkylreste eine paraffinische oder cykloparaffinische Struktur haben und 1 bis 10 Kohlenstoffatome pro Molekül enthalten,

b) Metallaryle eines Zusatzmetalls,

c) Metallalkyl-Aryle oder Metallaryl-Alkyle eines Zusatzmetalls, wobei die Alkylreste wie unter a) oben definiert sind,

d) Acetylacetonate eines Zusatzmetalls und

e) Metallsalze aus einem Zusatzmetall und organischen Säuren mit 1 bis 6 Kohlenstoffatomen pro Molekül.

9. Verfahren gemäß Anspruch 6, dadurch gekennzeichnet, daß die Metallverbindung gelöst im Reaktionslösungsmittel eingeführt wird und zwar vor Beginn der Einleitung des sekundären Alkohols.

10. Verfahren gemäß Anspruch 9, dadurch gekennzeichnet, daß man ferner gleichzeitig mit der Metallverbindung Wasserstoff einleitet und zwar in einer Menge von 0,5 bis 2 Liter pro Stunde und pro Gramm des Katalysators vom Typ Raney-Nickel.

11. Verfahren gemäß Anspruch 6, dadurch gekennzeichnet, daß die Einleitung der metallorganischen Verbindung gleichzeitig mit der beginnenden Einleitung des sekundären Alkohols in die Reaktionszone durchgeführt wird, wobei die metallorganische Verbindung im sekundären Alkohol gelöst ist.

12. Verfahren gemäß Anspruch 6, dadurch gekennzeichnet, daß die Menge des Zusatzmetalls 0,5 bis 4 Gew.-% beträgt.

13. Verfahren gemäß Anspruch 6, dadurch gekennzeichnet, daß man als Zusatzmetall Zinn verwendet.